# EUROPEAN PATENT APPLICATION

(11) **EP 1 269 866 A2**
(43) Date of publication of application: **02.01.2003**
(21) Application number: 02010421.2
(22) Date of filing: 08.05.2002
(51) Int. Cl.: A23L 3/32, A23L 3/16, A23B 4/015, A23K 1/10, A62D 3/00

(54) **Procedure and apparatus for making proteinaceous materials innocuous, in particular slaughter-house waste, at risk of transmitting infective prions**

(30) Priority: 21.06.2001 IT TO20010598
(71) Applicant: Officine Meccaniche Pejrani Srl, 10134 Torino (IT)
(72) Inventor: Morgantini, Gianpiero, Offici. Meccan. Pejrani Srl, 10048 Vinovo (IT); Pellegrin, Roberto, Officine Meccanic. Pejrani Srl, 10048 Vinovo (IT)
(74) Representative: Marchitelli, Mauro

(57) **Abstract**

A method for making proteinaceous materials innocuous, in particular slaughter-house waste. The treatment according to this method is susceptible to cause restructuring, into the alpha helical innocuous form, of the molecular structure of any infectious mutant form of prions present in the material subjected to treatment. Restructuring of the molecular structure is obtained through heat and cycles of electrical charges and discharges in the material.

A possible embodiment of an apparatus suitable to perform the aforesaid procedure is also described.

## Description

The present invention relates to a method and apparatus for making proteinaceous waste at risk of spreading infectious diseases of a prionic nature, innocuous, the method and apparatus being particularly suitable for the treatment of slaughter-house waste, in order to make these safe for use.

Until a few years ago, the scientific community deemed that infectious diseases could only be transmitted through agents with nucleic acid genomes. Recently, however, it was surprisingly discovered that specific infectious diseases, such as those referred to as "mad cow", can be transmitted by a single simple protein molecule with no genomes. It is in fact believed that the infectious agent of these particular diseases is a simple protein, the size of a thousandth part of a virus, nonetheless capable of causing modifications in the aggregation form of living matter.

This strange protein was called prion and is indicated by scientists with the initials PrP, from the abbreviation of "Prion Protein".

The prion is a small proteinaceous particle capable of withstanding procedures that modify nucleic acids without losing its infective capacity. Experience has shown that a prion cannot be modified by heat sterilization procedures or by ionising, radiation, ultraviolet radiation and, in general by treatments capable of degrading DNA and RNA to cause the death of the pathogenic spores and organisms.

Prion diseases, which at the current state of the art cannot be cured, are often called spongiform encephalopathies, as post-mortem shows the abnormal presence of vacuoles in the cortex of the brain and in the cerebellum. The most well-known diseases of this type are scrapie and BSE (Bovine Spongiform Encephalopathy) which sheep and cattle are subject to respectively. Diseases of the same type that affect other mammals are also known and the majority of mammals are probably subject to diseases of this type. The most well-known forms in humans are CJD (Creuzfeld-Jacob Disease) and FFI (Fatal Familial Insomnia).

Recently, following studies implemented to combat BSE, it was discovered that the cause of the diseases transmitted by prions was not to be found in the composition or sequence of the amino acids forming the prion protein it self, but in the spatial form in which the atoms are structured in the molecule.

Although maintaining the same chemical composition, the prion can have two forms which differ only in structure.

The first principal and stable form has the same regular helical structure as all normal proteins, namely the alpha helical structure. In this case the protein of the prion is innocuous and is indicated with the initials PrPc (common Prion).

Using X-ray diffractometry, it is however possible to detect that the protein taken from sick animals has a second form, differing slightly from the principal form and called beta form where, beside the principal groups of atoms regularly arranged in a helix, there are small groups arranged in a blade form. In this case the protein is distinguished with the initials PrPsc (scrapie Prion).

The PrPsc protein has moderate structural stability and it is believed that this is due to the presence of internal electric dipoles. Internal separation of the charges in fact causes the formation of electrostatic forces that can be stabilizing.

For a better understanding of the subject being dealt with, it is worthwhile recalling the way in which proteins are structured.

It is known that proteins are composed of long chains formed of amino acids bonded to one another by the peptide bond between the carboxylic group of one amino acid and the alpha amine group of the subsequent amino acid. Each amino acid participates in the peptide chain of the protein with the carbon of the carboxyl, the carbon bonded to it (alpha carbon) and with the nitrogen of the alpha amine group. In the chain the terminal carboxyl of one end and the terminal alpha amine group of the opposite end remain free.

The chemical architecture of the proteins is indicated by defining the respective primary, secondary and tertiary structures.

The primary structure relates to the chemical aspect, that is the sequence, fixed for each type of protein molecule, of the different types of amino acids bonded to one another to form the polypeptide chain, that is the framework of the molecule.

The secondary structure instead relates to the three-dimensional form. It is known that the proteins are arranged in a spiral in a stable structure called alpha helical through the effect of hydrogen bonds existing between the amide groups -CONH- of the same peptide chain (each amide group is also bonded by a hydrogen bridge to the third successive amide group) with atomic models that obligatorily comply with the atomic geometrical restrictions imposed by the angles formed by the bonds between the various atoms and by the interatomic distances.

However it is the tertiary structure that is particularly decisive for the stability of the proteins. It refers to the stabilizing bonds, such as the disulphuric bridges -S-S-, which lock the various spirals in their positions.

The secondary helical structure alone would not be sufficiently stable to withstand the ionic forces present in a solution. The proteins in living cells are in fact not solid but dissolved in intracellular fluid, so that the tertiary structure is essential to prevent breakup and randomly unwound chains from forming.

Recent literature states that prions are protein elements normally and physiologically present in living organisms and that there is nothing strange in the sequence of the amino acids of which they are formed, prevalently methionine and valine, and this is caused by a gene present in the chromosomes of the healthy mammals studied.

This in itself is not dangerous, as the prions are normally found in the ordinary structural form, the alpha helical form (PrPc) and are thus regularly metabolizable within the organism.

However, it is believed possible that, through rare cases of genetic mutation, part of the atomic structure of the protein PrPc can switch to another steric form, forming the protein PrPsc.

Studies seem to show that, while the protein PrPc can be attached by the protease enzyme, this does not occur in the protein PrPsc and this seems to be the cause of its characteristics of stability capable of cellular modifications.

It is believed that if the protein PrPsc is introduced into a healthy organism, for example through diet, after a certain period of incubation a chain reaction is triggered in which the mutant protein modifies the other proteins with which it comes into contact, causing their structure also to switch to the mutant form, and so on with exponential development.

In the light of this, the phenomenon of "mad cow" has been explained considering that in the last ten years, in order to increase the production of meat, livestock was imprudently fed on fodder containing animal meal and the disease was spread because the process used to produce these meals, as was subsequently established, was not able to make mutant proteins in the PrPsc form innocuous.

Animal meals are produced using a process called *rendering,* during which the carcasses of animals are first reduced to a pulp in water and the pulp is then heated to boiling and filter-pressed to separate the liquefied fat; the fatless pulp is then dried and pulverized.

To combat the symptoms of the "mad cow" phenomenon, it was initially deemed sufficient to adopt sterilizing conditions during rendering operations, believing that the conditions typically applied in sterilizing autoclaves (20 minutes at a temperature of 132°C produced by saturated steam at a pressure of 3 bar) would be sufficient to prevent the infectious disease from spreading.

However, as has been stated, while sterilizing treatments are capable of killing living organisms as they modify the nucleic acid of the proteins of which they are formed, it was observed that the same treatments are not capable of eliminating the cause of the disease BSE, deducing that the infectiousness is found in some other property not subject to the action of sterilizing treatments.

Having established the ineffectiveness of sterilizing heat treatment, the health authorities then decided to prohibit the use of animal meal in fodders and established the register of animals to control movements and origins, the destruction of certain parts of all sheep, goats and cattle, including the brain, spinal marrow, spleen, eyes, tonsils, etc., and the contact parts such as the bones of the cranium and the spinal column.

All these parts are in fact considered at risk even in animals that are apparently healthy, both due to the incubation period of the disease before its symptoms appear and to the impossibility of taking samples and testing the vital organs of live animals.

Following the aforesaid health provisions adopted at an international level to prevent recurrence of the phenomenon (which will probably be maintained even after regression of the spread of the disease), enormous quantities of slaughter-house waste have been created, the destruction of which causes problems of an operational and economic nature.

In the light of the above, the object of the present invention is to indicate a procedure and apparatus that allows treatment of slaughter-house waste, or proteinaceous materials, in order to rid them of any infectious prions.

This object is attained, according to the present invention, by a method and apparatus for making proteinaceous materials innocuous, in particular slaughter-house waste, at risk of transmitting infectious prions, with the characteristics claimed in the accompanying claims, which are an integral part of the present description.

Further objects, characteristics and advantages of the present invention shall become clear from the detailed description hereunder and from the accompanying drawings, provided purely as a non-limiting example, in which:
- figure 1 schematically represents, with a partly sectional side view, a possible embodiment of an apparatus for treating proteinaceous material operating in accordance with the present invention;
- figure 2 represents, in the form of a graph, the trend of the treatment temperature of the material (expressed in °C), as a function of the treatment time (expressed in minutes), for a complete operating cycle of the apparatus in figure 1.

As will become more apparent hereunder, in accordance with the procedure relating to the present invention slaughter-house waste or, more generally, proteinaceous materials subject to the risk of spreading infectious diseases of a prionic nature, are subjected to particular operating conditions, aimed at producing the conversion, to the innocuous alpha helical structural form, of any prions present in the infectious structural form.

For this purpose, in the procedure according to the invention the energy required to convert the proteinaceous molecule into the more stable alpha helical form is generated locally *in situ* at molecular level, through the kinetic energy provided by a bladed rotor, rotating at high speed inside a cell into which the slaughter-house waste or proteinaceous materials are loaded.

In a possible preferred embodiment, the apparatus for performing the procedure according to the invention comprises:
- a casing, preferably cylindrical in shape with a vertical axis, equipped with a cover and producing a closed cell electrically grounded, suitable to contain the material to be treated;
- a rotor, positioned inside the cell and preferably near the base of the latter, equipped with blades suitable for intense agitation of the particles of the material being treated;
- means susceptible to keep the aforesaid rotor, its control shaft and its support electrically insulated from the cell and from the ground;
- means to control rotation of the rotor at a variable speed, so as to generate through impact a high temperature directly inside the particles of the material being treated, and surface charges of static electricity through rubbing between the surfaces of the particles in a totally dry state;
- means for controlled supply of water inside the mass of material being treated;
- means for washing the cell with air to extract the steam produced therein;
- means to remove or scrape the material from the side walls of the cell;
- means to control the temperature, acting on the quantity of water dosed and on the rotation speed of the rotor;
- means for absorption in water of the steam drawn outside the cell by the flow of washing air;
- means to feed the material to be treated into the cell;
- means to maintain a vacuum in the cell.

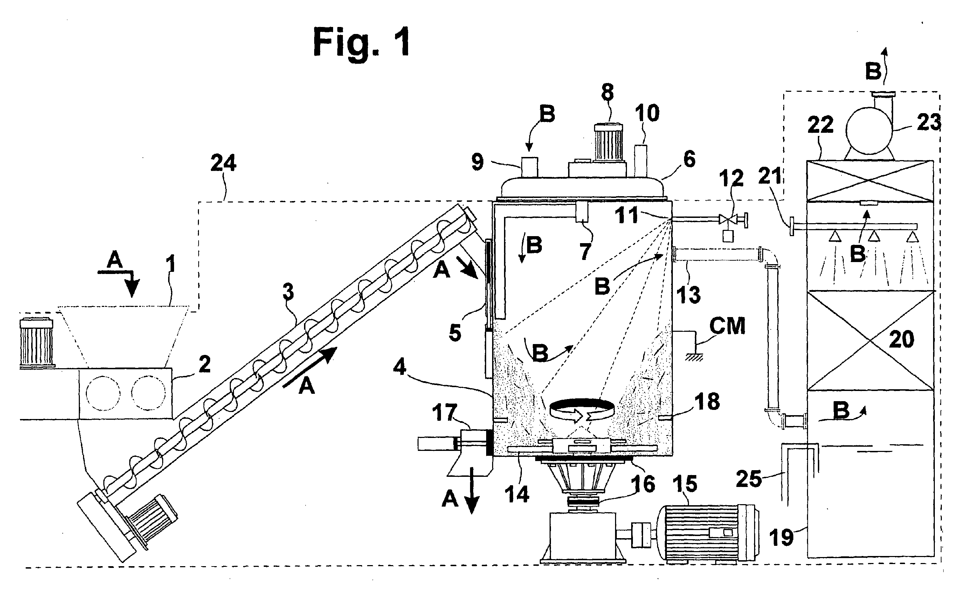

Figure 1 indicates, as a non-limiting example, a possible embodiment of an apparatus for performing the procedure according to the invention.

In this figure, the number 1 indicates a hopper, destined to receive the proteinaceous material to be treated, such as slaughter-house waste; 2 indicates a triturator of a known type, provided to crumble the material delivered from the hopper 1; 3 indicates an auger conveyor, provided to receive the crumbled material from the triturator 2 and convey it towards the inside of a cylindrical treatment chamber or cell, indicated as a whole with 4; for this purpose, the upper part of the cell 4 is provided with an inlet for the material to be treated, in line with which a motor-driven shutter is fitted, indicated with 5, for the purpose of shutting off, at the end of loading, the feed system from the cell 4.

The cell 4, which is made of steel sheet, is closed at the top with a cover 6, which carries a scraping system 7 for the internal walls of the cell, of known design, operated by a gear motor 8 positioned on the outside of the cover.

Also installed on the cover 6 are an inlet 9 for the washing air and a system 10 for measuring the temperature of the infrared rays, per se known.

Positioned in the top part of the cell 4 is a nozzle 11 for supplying water to the cell, controlled by a dosing solenoid valve 12 located on a respective water supply line.

Also positioned in the top part of the cell 4 is an outlet for the washing air, connected to a respective suction line indicated with 13.

Positioned in the lower part inside the chamber 4 is a bladed rotor 14, which is operated by a motor-driven system, indicated as a whole with 15, and comprising a motor, a power transmission system and a support for the rotor.

The cell 4 is electrically grounded, by means of a ground contact indicated with CM, while the rotor 14 and the respective support and drive shaft are electrically insulated from the cell and from the other parts of the apparatus, by means of known insulating materials indicated schematically with 16.

Also located in the lower part of the cell 4 is a motor-driven valve 17 which opens to allow unloading of the treated material at the end of the cycle; this material is removed from the cell through centrifugal force.

Also positioned in the lower part of the cell 4 are fixed blades, integral with the side walls of the cell, one of which is indicated with 18, designed to operate in combination with the moving blades of the rotor 14.

The number 19 indicates as a whole a column for treating the steam which is removed from the cell 4 by means of a suction line 13.

In particular, the column 19 has the function of purifying the air utilized to wash the cell and absorbing the steam transported by it on a pack of filling bodies, indicated with 20, by means of a flow of cold water sprayed from above through a series of nozzles 21.

The number 22 indicates a filter containing activated carbon, designed to withhold the odours, through which the air delivered from the pack 20 travels, before being expelled into the environment through an exhaust fan 23, which maintains a constant vacuum in the apparatus.

For this purpose, in the preferred embodiment, the apparatus according to the invention is produced in single piece, with the various aforesaid components closed inside a case, indicated with 24, for example produced by a framed structure.

The number 25 indicates an overflow with water seal of the known type, through which the used water, previously sprayed through the nozzles 21, is conveyed towards a drainage system.

Finally, in figure 1 the arrows marked with the letter "A" identify the route of the material being treated, while the arrows marked with the letter "B" indicate the route of the air flow.

The apparatus in figure 1 operates in the following manner.

The material to be treated is loaded into the hopper 1, to be crumbled by the triturator 2 and then conveyed by the conveyor 3 towards the cell 4; for this purpose, the shutter 5 is opened, to allow the conveyed material to enter the inlet to the cell 4, and subsequently closed.

The material then falls onto the bottom of the cell, where the rotor 14 is made to rotate at high speed; indicatively the rotor speed varies from 1,000 to 3,000 rpm.

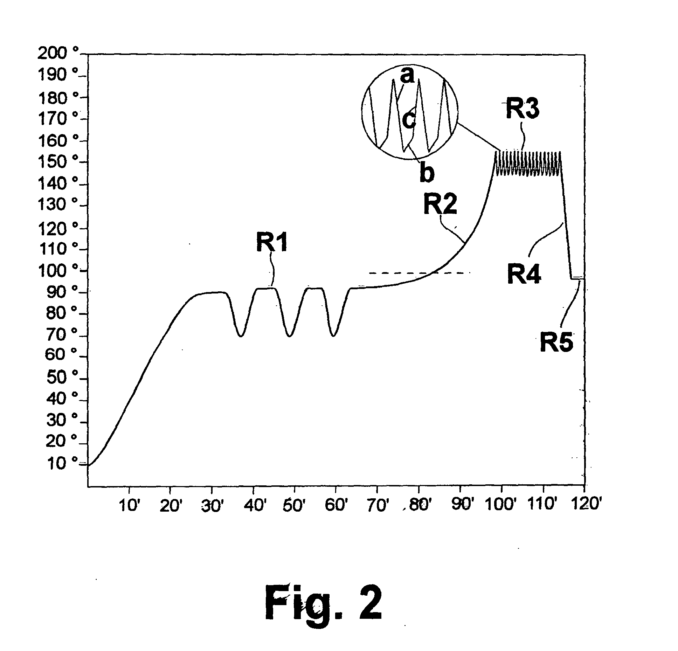

The graph in figure 2 represents, for one complete treatment cycle in the cell 4, the temperature trend in °C of the material, as a function of the treatment time in minutes.

During the initial step R1, heat is generated inside the particles of material, through the impact caused by the blades of the rotor 14, this heat causing total evaporation of the water contained in the material being treated.

At the end of step R1, which in the case exemplified in the graph in figure 2 lasts for about 80-90 minutes, the mass of material reaches in the aforesaid manner approximately 100 °C and becomes totally dry. It must be pointed out that while the aforesaid temperature is being reached during step R1, as the material becomes dehydrated inside the cell 4, thus progressively losing water and weight, it is replenished with fresh material (through the system formed of the hopper 1, triturator 2, conveyor 3 and shutter 5); in this way an optimum load is reestablished for rotor 4 operation. Therefore, as can be seen in the graph in figure 2, each time material is loaded, there is a temporary drop in the temperature, which subsequently gradually rises again as dehydration of the material is completed.

After the last material loading phase (which in the case exemplified in figure 2, occurs about 60' from the beginning of the treatment cycle) and after all the humidity has been eliminated upon exceeding at temperature of 100°C, in the subsequent step R2 impact and rubbing between the dried particles, again through the action of the rotor 14, provide these particles with electrostatic charges while, simultaneously, the temperature rises to a programmed value, selected in the range from 130°C to 170°C, preferably around 150°C.

In the subsequent step R3 there are periods each lasting for about one minute, in which three respective phases alternate. Again with reference to the graph in figure 2, the enlarged part shows a first phase indicated with the letter "a", in which water is delivered to the cell through the nozzle 11 and the solenoid valve 12; the temperature then drops suddenly from approximately 150°C to approximately 140°C and the particles of material become conductive and discharge the electrostatic charge accumulated to the ground, to which the cell 4 is connected by means of the contact CM.

Again with reference to step R3, a second phase, indicated with the letter "b", follows in which the dosed water evaporates and the material being treated returns to the totally dry state.

Finally, this is followed by a third phase, indicated with the letter "c", in which the dry material is returned to the programmed temperature of around 150°C, while its particles are recharged electrically. In this phase the charges are not dispersed to the ground, through an effect caused by the deposit of dry material on the internal walls of the cell 4, which acts as an adequate insulating material.

In order for the steam emitted during phase "b" to be easily eliminated, so as to leave the environment dry to allow phase "c to take place regularly, the cell 4 is kept constantly in a vacuum through the action of the exhaust fan 23 (and consequently of the suction line 13 and the column 19), which supplies new washing air to the cell 4 through the specific inlet located on the cover 6.

This air then washes the cell 4, removing the steam that has formed there, through the line 13, for subsequent treatment in the column 19, with the previously described method.

The quantity of water delivered from the nozzle 11 during step R3 is preferably 20 ml for each kg of dry material being treated; moreover, as will be clarified hereunder, this dosage is automatically controlled by the system 10, which allows the temperature to fluctuate exactly within the programmed range.

By analysing in detail what occurs during step R3, it can be seen that in the first phase "a" of each period the dosed water is distributed on the surfaces of the cell membranes of the material being treated; this causes localized cooling of the surfaces of the particles, which is promptly detected by the infrared emission measurement system 10. The water is then rapidly absorbed by the totally dehydrated cell membranes of the particles of material, penetrating inside.

In the second phase "b" heat, generated continually inside the particles through the effect of impact between these caused by the rotor 14, causes rapid evaporation of the water absorbed and the breakage, though increase in internal pressure, of the cell membranes which have also been weakened by the hydrolytic reaction of the proteins with water. This makes the plasmatic material inside and the polypeptide chains of the prions progressively accessible.

During phase "c" the material is taken to a temperature that causes considerable movement of atoms, together with an electrostatic charge; this combination is capable of eliminating the dipoles inside the molecule in the subsequent discharge phase "a", with the molecule being restructured into the alpha helical structural form, stable and electrically neutral inside.

The quantity of water added through the nozzle 11 and the relative dosing solenoid valve 12 in each period is controlled automatically by the instant temperature measurement system 10, which operates by reading the infrared emission radiated from the material, so that the programmed temperature fluctuation is obtained and the temperature is simultaneously prevented from rising or dropping excessively. The information drawn from this system 10 is also used to automatically control the rotation speed of the rotor 14, in order to balance the quantity of water dosed with the quantity of energy discharged on the particles of material being treated.

Treatment of the material according to step R3 continues for a period sufficient for probabilistic completion of structural reconversion; as an indication, a time of about 10 minutes may be considered efficacious.

In order for all the material to be subjected to the same treatment, when necessary the scraper system 7 returns the particles adhering to the walls of the cell 4 to the zone influenced by the rotor 14.

After the treatment time according to step R3, during the subsequent step R4 the mass of material is cooled by further dosing of water through the nozzle 11, which brings the temperature to approximately 100°C.

In the subsequent step R5 the material is extracted by centrifugal force from the cell 4, in the form of dry granules, by opening the valve 17, and is then left to cool.

The dry granules, the molecule of which has now been returned to the alpha helical structural form, have thus become innocuous, safe to handle and usable, for example to produce fertilizers.

A further advantage of the procedure is represented by the fact that, as it is totally dehydrated, the weight of the treated material is reduced to a third of the initial weight; as the treated material is dry, it is also stable and can be stored for lengthy periods.

The characteristics of the method and the apparatus for making proteinaceous materials innocuous, in particular slaughter-house waste, at risk of transmitting infectious diseases of a prionic nature, according to the present invention, which are further detailed in the accompanying claims, are apparent from the above description.

The advantages of the invention are also apparent from the description. In particular, it must be once again emphasized how the solution described allows the molecular structure of any prions present in the infectious mutant form to be restructured into the innocuous alpha helical form, in order to make the treated material safe to handle and use.

## Claims

1. Method for making proteinaceous materials innocuous, in particular slaughter-house waste, where making innocuous is based on the restructuring or conversion, into the innocuous alpha helical form, of the molecular structure of any infectious mutant form of prions present in the material being treated.

2. Method according to Claim 1, **characterized in that** said restructuring is induced through heat in combination with electrostatic charge and discharge cycles, and elimination of the electric dipoles inside the molecule.

3. Method according to Claim 2, **characterized in that** the electrostatic charging phase is produced by rubbing between particles of the material being treated in a totally dry state.

4. Method according to Claim 3, **characterized in that** the electrostatic discharge phase is performed by dampening and making conductive the particles of material previously subjected to electrostatic charging.

5. Method according to Claim 2, **characterized in that** heat is generated *in situ* inside the particles of the material being treated through the effect of impact and rubbing produced between these particles.

6. Method according to Claim 5, **characterized in that** said impact is caused by the action of rotor means (4), in particular bladed, which are made to move at high speed in the mass of material being treated.

7. Method according to Claim 6, **characterized in that** the high speed movement of said rotor means (14) produces rubbing between said particles which, in the periods in which the material being treated is in the dry state, causes these particles to be charged with electrostatic energy.

8. Method according to any of the preceding Claims, **characterized in that** water is added to the material being treated, in particular in a periodic and dosed manner.

9. Method according to Claim 8, **characterized in that**, in addition to the action of electrostatic discharge, the water also interacts with the cellular membranes of the material being treated, in order to damage these through the effect of the hydrolytic reaction caused by the water, in the liquid state, in contact with the proteinaceous substance heated to a temperature considerably higher than the boiling point of water at ambient pressure.

10. Method according to any of the preceding Claims, **characterized in that** the water interacts with the organic substance present in the material being treated, with the water first being absorbed inside the totally dried cells and subsequently transformed into steam with increase in the internal pressure of the cells and breakage of the cell membranes.

11. Apparatus for implementing the method according to any of the preceding Claims.

12. Apparatus according to Claim 11, **characterized in that** it comprises:
- a casing forming a cell (4), suitable to contain and treat proteinaceous material, particularly slaughter-house waste, electrically grounded;
- a rotor (14) positioned in the lower part inside the cell (4), equipped with blades suitable for intense agitation of the particles of the material;
- means (16) suitable to keep the aforesaid rotor (14) and relative supporting and operating means electrically isolated from the ground, from the cell (4) and from the other parts of the apparatus;
- means to control rotation of the rotor (14) at a variable speed, so as to generate heat through impact directly inside the particles and surface charges of static electricity through rubbing between the surfaces of the totally dried particles;
- means (11, 12) for controlled delivery of water inside the mass of material being treated;
- means (23) to maintain a vacuum in the cell (4, 9, 13), forming a flow of washing air,
- means for scraping (7) the side walls of the cell (4), suitable to keep the material in the zone influenced by the rotor (14),
- a system to control (10) the lower and upper temperature thresholds, through real time measurement of the infrared emission radiated from the material, said system acting on the quantity of water dosed in the material with adjustment of the rotation speed of the rotor (14) possible.

13. Apparatus according to Claim 12, **characterized in that** it comprises means to feed the material to be treated, comprising a hopper (1), a triturator (2), a conveyor (3) and a shutter (5).

14. Apparatus according to Claim 12, **characterized in that** it comprises a system for treating (19) the air flow made to travel through the cell (4) with water.

15. Apparatus for making proteinaceous materials innocuous, in particular slaughter-house waste, **characterized in that** it comprises a layout susceptible to induce restructuring or conversion, into the innocuous alpha helical form, of the molecular structure of any infectious mutant form of prions present in the material being treated.

16. Apparatus according to Claim 15, **characterized in that** said layout comprises means (14) to induce heat in particles of the material being treated.

17. Apparatus according to Claim 15, **characterized in that** said layout comprises means (8-14) to produce electrostatic charge and discharge cycles in particles of the material being treated.

18. Apparatus according to Claim 15, **characterized in that** said layout comprises means (8-14) to eliminate the electric dipoles inside molecules of particles of the material being treated.

19. Apparatus according to Claim 15, **characterized in that** said layout comprises means (11, 12) to humidify and/or make conductive the particles of the material being treated previously subjected to electrostatic charging.

20. Apparatus according to Claim 15, **characterized in that** said layout comprises means (14, 18) to produce reciprocal rubbing between particles of the material being treated.

21. Apparatus according to one or more of Claims from 15 to 20, **characterized in that** it comprises:
- a chamber or cell (4) to contain the material to be treated, electrically grounded, said cell (4) being in particular cylindrical in shape with a vertical axis;
- rotor means (14) operating inside said cell (4), in particular near the base of the latter, suitable to produce intense agitation of particles of the material being treated;
- insulating means (16), susceptible to keep said rotor means (14) electrically insulated from the cell (4) and from the ground;
- means (11, 12) for the controlled supply of liquid, in particular water, to said cell (4);
- means (9, 13, 19, 23) for washing said cell (4) with air, in order to extract the steam produced there;

22. Apparatus according to Claim 21, **characterized in that** it is also provided with means (10, 15) to control rotation of said rotor means (14) at a variable speed.

23. Apparatus according to Claim 21, **characterized in that** it is also provided with means (7) to remove or scrape the material being treated from the walls of said cell (4).

24. Apparatus according to Claim 21, **characterized in that** it is also provided with means (10) to control the temperature, acting in particular on the quantity of liquid dosed and the rotation speed of said rotor means (14).

25. Apparatus according to Claim 21, **characterized in that** it is also provided with means (13, 19, 23) to draw steam outside said cell (4), and for any subsequent purification of this steam.

26. Apparatus according to Claim 21, **characterized in that** it is also provided with means (1-3, 5) to feed the material to be treated inside said cell (4).

27. Apparatus according to Claim 21, **characterized in that** it is also provided with means (23) to maintain a vacuum in said cell (4).
